Europäisches Patentamt

European Patent Office

Office européen des brevets

(19)

(11) Publication number: **0 330 510**

A2

(12) **EUROPEAN PATENT APPLICATION**

(21) Application number: 89301886.1

(22) Date of filing: 24.02.89

(51) Int. Cl.⁴: **C 07 C 53/10**
// B01J2/04

(30) Priority: 24.02.88 GB 8804337

(43) Date of publication of application:
30.08.89 Bulletin 89/35

(84) Designated Contracting States:
BE DE ES FR GB IT NL

(71) Applicant: Albright & Wilson Limited
210-222 Hagley Road West
Oldbury Warley West Midlands B68 0NN (GB)

(72) Inventor: Smith, Raymond Anthony
28 Woodside Close
Walsall West Midlands WS5 3LU (GB)

James, Michael Leslie
4 Cambridge Road Hensingham
Whitehaven Cumbria CA28 8 NU (GB)

(74) Representative: Wilson, Michael John et al
c/o Albright & Wilson Limited 1 Knightsbridge Green
London SW1X 7QD (GB)

(54) Acetate product and process.

(57) Hydrated sodium acetate beads usually have a core and shell structure, the shell comprising anhydrous sodium acetate and the core hydrated sodium acetate. The beads may be made by prilling to form an intermediate body with solid shell and solid/liquid core prior to further cooling.

EP 0 330 510 A2

**Description**

## ACETATE PRODUCT AND PROCESS

This invention relates to acetate compositions, in particular ones comprising hydrated sodium acetate, and processes for making them.

Sodium acetate can exist in the form of the crystalline trihydrate whose crystals are sticky and do not flow freely, and in the form of the anhydrous material which is a fine powder and therefore causes dust problems. We have now discovered a hydrated sodium acetate which can have reduced caking problems and improved flow properties but not cause dust problems.

The present invention provides beads of hydrated sodium acetate usually with a molar ratio of water to sodium acetate of 2.5-3.2:1 e.g. 2.8-3.2:1. Advantageously the beads have an outer layer comprising anhydrous sodium acetate and a core containing sodium acetate trihydrate, especially when the molar ratio of water to sodium acetate is less than 3:1 e.g. 2.5-3.0:1 such as 2.8-3.0:1 or particularly 2.7-2.95:1.

The present invention also provides a process for preparing beads of hydrated sodium acetate, wherein an aqueous solution at 70-120°C of sodium acetate with a molar ratio of water to sodium acetate of 3.0:1 to 3.5:1 is formed into droplets which are rapidly cooled substantially out of contact with one another by counter current contact with air to produce an intermediate body with a solid shell and a liquid core, and this is then subsequently cooled to produce beads of hydrated sodium acetate. Particularly when the water to sodium acetate molar ratio in the solution is 3-3.2:1, the rapid cooling produces particles of an intermediate body with a solid outer layer of anhydrous sodium acetate enclosing a core containing aqueous medium comprising sodium acetate and said particles of intermediate body are cooled further to give the solid beads of hydrated sodium acetate, usually with a molar ratio of water to sodium acetate of 2.5-3.0:1 e.g. 2.8-3.0:1.

Preferably the particles of the intermediate body are separated from said counter current air before the subsequent cooling, though at the separation stage the core usually contains solid sodium acetate as well as said aqueous medium.

In the hydrated solid acetate of the invention, with the core shell structure the molar ratio of water to sodium acetate is not constant throughout the solid, because it is zero or slightly above, in the outer layer and 3:1, or slightly below, in the inner core; the thickness of core and outer layer are such as to give the desired overall water to sodium acetate ratio. The core/outer layer structure may be shown by X-ray crystallographic analysis of samples taken across a diameter of the particles.

The solid composition of the invention is substantially completely water soluble at 20°C. While the solid compositions usually consist essentially of sodium acetate and water, they may contain small amounts, e.g. up to 1%, especially up to 0.5% (by weight based on the weight of sodium acetate) of sodium carbonate, either present as an intentional or accidental ingredient in the sodium hydroxide solution used in the production of the aqueous solution being solidified or because of absorption by the droplets of carbon dioxide from the air used in the solidification process.

The solid compositions of the invention are particles obtained by ejecting droplets of the hot aqueous solution from a sprayer into a stream of gas moving countercurrent to the droplets which subsequently continue to move in countercurrent to the air and essentially out of contact with each other until the beads are produced or until the intermediate body with the solid outer layer or shell of anhydrous sodium acetate and core containing liquid is produced. After separation if desired from the countercurrent air flow, the particles of this intermediate body are allowed to cool further in or out of contact with each other until the body solidifies completely and its temperature is less than 30°c. The gas initially contacting the droplets as they emerge from the sprayer is at a temperature less than that of the droplets, preferably at less than 60°C e.g. at 30-50°c and that finally contacting the partly solidified intermediate body with solid shell and liquid or liquid/ solid core is also at less than the temperature of that body, e.g. at 0-30°c such as at 10-20°C. Advantageously the droplets during solidification contact gas of progressively reduced temperature e.g. as happens when the droplets fall under gravity in countercurrent to an upward stream of gas e.g. air, the air being cold e.g. 0-20°C at the bottom and being warmed progressively by the molten droplets passing through it. This solidification may be performed in a prill tower in which the hot aqueous solution is pumped to the top of the tower and under sufficient pressure to atomize it, the solution is ejected from one or more, e.g. 1-6 or 2-5, nozzles in a spray head in the form of droplets which fall down the tower against an upward flow of air. The nozzles may be of the solid cone or the hollow cone type and are arranged to minimize overlap between the sprays of droplets and to minimize impact of the droplets on the walls of the tower and to maximize spraying of droplets down the tower. The partly solidified body with the solid shell and liquid or liquid/solid core may be collected from the bottom of the tower and subsequently cooled. The ejection of hot solution into the gas usually causes evaporation of some of the water in the solution, thereby decreasing the water:sodium acetate ratio from 3-3.5:1 to 2.5-3.0:1 or 2.8-3.2:1.

The higher the concentration of sodium acetate in the hot aqueous solution the higher the temperature of spraying. Thus with the hot solutions of 56-58.7% sodium acetate, spray temperatures of 70-90° C may be used while for solutions of 58.7-61% sodium acetate, temperatures of 90-120°C are preferred.

In the process there is initial cooling of the droplets to cause surface solidification while at the same time there is evaporation of water from the solution to reduce its water to sodium acetate ratio.

The gas initially contacting the hot droplets preferably has a Relative Humidity of 10-90%. If the hot solution already has a water to sodium acetate ratio of 2.8-3.2:1, eg. 3-3.2:1, then the gas may be saturated with water vapour, but advantageously hot sodium acetate solutions of water to sodium acetate ratio of 3.0-3.5:1 are used and the water to sodium acetate ratio is reduced in the spraying and solidification steps; in order to obtain products of the desired structure it is then essential that water vapour is evaporated from the droplets before the solidification of the outer shell is complete, as happens when the vapour pressure of the water in the gas contacting the hot molten droplets is less than the vapour pressure of water over the droplets at the temperature of the droplets. When the inlet gas has a high relative humidity and a higher temperature, it is capable of effecting less cooling and less decrease of the water: sodium acetate ratio of the particles than with colder inlet gas of lower relative humidity. The flow of droplets and the $H_2O$:sodium acetate content of the input liquid may be adjusted during continuous operation to compensate for variations, if any, in temperature and humidity of the air and to produce a solid product, with the higher the air inlet temperature at constant air flow rate the lower the liquid flow rate, and the higher the humidity the higher the sodium acetate $H_2O$ ratio of the input liquid. In addition the higher the flow rate of the air, the higher can be the flow rate of the liquid being solidified; there may be used ratios of the volume of liquid per hour to the volume per hour of gas, e.g. air of not more than 1:10, e.g. not more than 1:15 or 1:20 such as 1:10-50 or 1:15-30.

The intermediate body has a solid shell which can be of anhydrous compound if the overall body has less than 3:1 water to sodium acetate and a core which contains a liquid phase of sodium acetate trihydrate. The core usually also contains solid trihydrate so the core may have partly, e.g 50-90%, solidified but not completely solidified. The intermediate body is usually at 30-70°C, e.g. 40-70°C or 30-50°C, these temperatures being the measured average for the body because it has a relatively cold solid shell and a relatively hot core containing the crystallizing liquid phase and usually solid phase as described above. The intermediate body has sufficient strength that the particles do not coalesce on contact wth one another.

The production of the intermediate body is usually performed in a prill tower of height at least sufficient so that the intermediate body has sufficient strength not to break when hitting the prill tower bottom. The tower is usually 10-30M high, e.g. 12-20M. The warm intermediate product is then cooled further to below 35°C, e.g. to below 30°C. The cooling may occur in the tower but preferably is outside the tower. Thus the intermediate body may be cooled at the bottom of the tower by passing air countercurrent through it. This cooling could occur while the particles of intermediate body fall under gravity out of contact with one another in a tower, e.g. with the droplets of hot liquid being sprayed down a tower 50-70M long against a countercurrent stream of air to produce at the bottom a completely solid composition of the invention at below 30°C. Alternatively a shorter tower could be used so long as the droplets fall out of contact with one another until the intermediate body is produced and then the particles of intermediate body, a significant proportion of which are in contact with one another, e.g. in the form of spheroids with semi molten centres, are cooled by passing air countercurrent through them, before they leave the tower at the bottom at below 30°C.

Complete cooling in the tower inevitably raises the temperature of the air contacting the droplets at the top of the tower. Therefore it is preferred to cool the intermediate body outside the tower. The intermediate body is advantageously separated from the counter current air stream and is subsequently cooled outside the tower. This later cooling can take place with at least a portion of the particles in contact with one another, e.g. when air is passed through a mass of particles of the intermediate body or is passed through or over a moving optionally perforated conveyor or table on which the intermediate body lies. However the intermediate body is preferably significantly further cooled with the particles substantially out of contact with one another. Thus the particles of intermediate body may be passed from the tower, e.g. by conveyor or vibrating table, into a chamber where they are mixed with air, e.g. tumbled with air and then separated in a cyclone. Most preferably the particles of intermediate body are passed from the tower into a second tower or conduit where they are contacted cocurrent with air having a temperature of 0-30°C e.g. by being passed into an upwardly moving air stream in which they move cocurrently and are cooled to 15-30°C, e.g. in an air lift.

If desired, before the second cooling outside the tower, any oversize particles may be removed. The cooled product of the invention is then obtained of substantially uniform particle size with few fine materials, in contrast to the product obtained by spray drying which gives particles with a breadth of sizes including many fines, e.g. at least 50% of less than 0.25mm and at least 10% and often at least 20% of less than 0.075mm. The process can therefore give a higher yield of product of substantially uniform size, e.g. greater than 0.18mm, than spray drying. Any fines made in the process of invention may if desired be removed and for example redissolved in the solution of sodium acetate prior to the formation of droplets.

The particles of product of the invention, which are usually substantially spherical, are preferably of 0.1-2mm, e.g. 0.25-1.5mm; at least 95% may be of 0.18 to 1.5mm. The vast majority, e.g. at least 90% by weight are usually of 0.5-1.5mm and a large majority, e.g. at least 80% are of 0.7-1.5mm, e.g. about 1.0mm diameter. The particles of product usually contain less than 1% of less than 0.1mm. The particles of product of the invention are usually obtained from hot droplets of substantially the same size sprayed from nozzles which are 0.7-4.0mm e.g. 1.6-3.6 or about 2.5mm in diameter.

The aqueous feed solution to the sprayer which is usually of 56-61% sodium acetate concentration may conveniently be made by reacting a concentrated aqueous solution of acetic acid or partly sodium neutralized acetic acid with the requisite amount of concentrated sodium hydroxide solution to give the solution of sodium acetate. The neutralization of the solution is exothermic and the hot solution may advantageously be used as such for the solidification process of the invention, though extra heat may be provided if required in order to keep the solution of that concentration liquid before the solidification. The aqueous solution before or after the final neutralization may be concentrated if required before use in the process.

The solid acetate compositions of the invention have a reduced tendency to cake, and flow more freely than the crystalline products and have less tendency to dust than the anhydrous material. The products of the invention with the anhydrous outer layer and hydrated core e.g. with water to sodium acetate molar ratio of 2.7-2.9:1 tend to flow more freely still than those with the molar ratio at 3.0:1 or more. The products of the invention may be used as weak bases for a wide variety of purposes such as for use as additives in textiles and dye stuff processing, or in the production of pharmaceutical compounds.

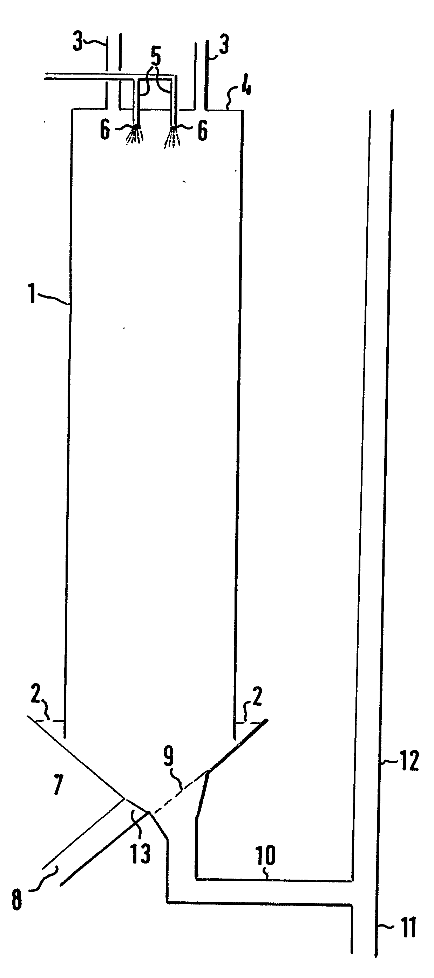

The process may be performed in apparatus as illustrated in the accompanying figure which is a schematic representation of a prilling tower with associated pipework.

Tower 1 has inlets, 2, for air at its bottom and exits, 3, for warm air in its top, 4. Also fitted in its top, 4, are two inlet pipes, 5, with four fixed spray nozzles, 6, through which hot liquid may be ejected. The tower has an inverted conical bottom, 7, the inside surfaces of which, together with the end of the vertical sides of the tower, define the air inlets, 2. On one side of the conical bottom is an exit, 8, for any large lumps with a movable door, 13, and on the other side of the conical bottom, 7, is a grating, 9, e.g. of 30mm spacing for removal of particles of product. The particles pass through grating, 9, down line 10 which may contain conveying means, e.g. a vibrating table or screen, and are then separated by gravity into any residual large bodies which fall down line 11 and desired particles which move up line 12 carried by a cocurrent stream of air, which enters line 11 and passes up line 12. A suction fan and cyclone (not shown) may be attached to the upper end of line 12 finally to separate the particles of product from the air.

In use the hot aqueous solution of sodium acetate is sprayed down the tower 1 in countercurrent to cooler air passing up the tower from inlet 2 to exits 3. The liquid is partly solidified to give intermediate product with solid shell and partly solidified core and the intermediate product is separated at the bottom of the tower by the grating, 9, into large lumps which are retained and the rest of the product which passes through the grating and is cooled and separated further as described above to give completely solidifed particulate product.

The lumps, which are agglomerations of particles and/or material from the tower walls, fall off the grating, 9, and periodically are removed down exit line 8 by opening the door 13. The air leaving exits 3 may contain ultra fine dust particles of the solid, whose phosphate values may be recovered by scrubbing (not shown).

The process is illustrated in the following Examples in which the solidification apparatus was a simplified version of the apparatus shown in the accompanying Figure, which was otherwise used in a manner generally as described above. In the apparatus there was a column 1 15m high and 1m diameter with an inlet region 2 at its bottom for input air and an exit region 3 over its top for warm air; the column was not enclosed at the top. Into the open top of the column was an inlet pipe 5 with four fixed spray nozzles 6 for spraying the liquid. Beyond the open bottom of the column was a collection container (not shown in the Figure) for collecting the solid product.

In Example 1 a 58% aqueous solution of sodium acetate whose pH diluted to 10% solution was 9 having a water to sodium acetate ratio of 3.3:1 was obtained at 75-80°C and sprayed in the form of droplets down the column in counter current to cooler air passing up the column by convection from inlet region 2 to outlet region 3. The droplets cooled to give an intermediate body with a shell of hydrated sodium acetate and a liquid core, and then cooled further to give at the bottom of the column solidified prills or beads of hydrated sodium acetate having a water to sodium acetate molar ratio of 3.0:1. The beads were of 1.0mm average particle size with 90% greater than 0.15mm size and more free flowing than crystalline sodium acetate trihydrate. In comparative tests the angle of repose (a measure of freedom to flow) was 45° for the latter crystals and 30° for the beads.

## Example 2

The process of Example 1 was repeated in Example 2 with a hot aqueous solution of 59.5% sodium acetate solution (having a water to sodium acetate molar ratio of 3.1:1) which was sprayed at 105°C to give warm beads, which were more free flowing than those in Example 1 (angle of repose 25°).

The beads of 1.0mm average particle size with 90% greater than 0.15mm size, had a shell of anhydrous sodium acetate and a core of sodium acetate trihydrate (based on Xray analysis) and had a water to sodium acetate molar ratio of 2.9:1.

## Claims

1. A solid hydrated sodium acetate bead.

2. A bead according to claim 1 of particle size 0.1-2mm.

3. A bead according to claim 1 or 2, wherein the overall molar ratio of water to sodium acetate is 2.8-3.0:1.

4. A composition according to any one of claims 1 to 3 having an outer layer comprising anhydrous sodium acetate and a core containing sodium acetate trihydrate.

5. A process for preparing solid hydrated sodium acetate beads, wherein an aqueous solution at 70-120°C of sodium acetate, with a water to sodium acetate molar ratio of 3-3.5:1 is formed into droplets and rapidly chilled substantially out of contact with one another by countercurrent contact with air to produce an intermediate body with a solid shell and liquid core, and this body is then cooled to produce beads of hydrated sodium acetate.

6. A process according to claim 5 wherein an aqueous solution at 90-120°C of sodium acetate containing water to sodium acetate in a molar ratio of 3-3.2:1 is formed into droplets and rapidly chilled substantially out of contact with one another by countercurrent contact with air to produce particles of an intermediate body with a solid outer layer comprising anhydrous sodium acetate enclosing a core comprising aqueous medium comprising sodium acetate and then said particles of the intermediate body are cooled further to give a solid acetate composition having a solid outer layer comprising anhydrous sodium acetate and a solid core containing sodium acetate trihydrate.

7. A process according to claim 6 wherein particles of the intermediate body are separated from said countercurrent air before being cooled further.

8. A process according to claim 6 or 7 wherein droplets of 0.1-2mm size initially contact the countercurrent air, which has a temperature of less than 60°C, the particles of intermediate body have an average measured temperature of 70-30°C and are cooled to give the solid beads at less than 30°C.

9. A process according to any one of claims 6-8 wherein the particles of intermediate body are cooled further substantially out of contact with one another.

10. A process according to claim 9 wherein droplets of 0.25-1mm of said aqueous solution pass down a tower countercurrent to an upwardly moving air stream to give particles of intermediate body having an average temperature of 35-50°C, said particles are then separated from said air stream and passed into an upwardly moving air stream in which they move concurrently and are cooled to 15-30°C to produce said solid acetate beads.